**Europäisches Patentamt**

(19) **European Patent Office**

**Office européen des brevets**

(11) Veröffentlichungsnummer : **0 057 884 B2**

(12) **NEUE EUROPÄISCHE PATENTSCHRIFT**

(45) Veröffentlichungstag der neuen Patentschrift :
**15.04.92 Patentblatt 92/16**

(51) Int. Cl.⁵ : **C07C 209/00,** C07C 211/13,
C07C 215/00, C07C 217/04,
C07C 211/14

(21) Anmeldenummer : **82100669.9**

(22) Anmeldetag : **30.01.82**

(54) **Verfahren zur Herstellung von permethylierten Oligoaminen und Aminoalkanolen.**

Die Akte enthält technische Angaben, die nach
dem Eingang der Anmeldung eingereicht
wurden und die nicht in dieser Patentschrift
enthalten sind.

(30) Priorität : **11.02.81 DE 3104738**

(43) Veröffentlichungstag der Anmeldung :
**18.08.82 Patentblatt 82/33**

(45) Bekanntmachung des Hinweises auf die
Patenterteilung :
**02.04.86 Patentblatt 86/14**

(45) Bekanntmachung des Hinweises auf die
Entscheidung über den Einspruch :
**15.04.92 Patentblatt 92/16**

(84) Benannte Vertragsstaaten :
**BE DE FR NL**

(56) Entgegenhaltungen :
**EP-A- 0 006 454**
**DE-A- 2 709 864**
**DE-A- 2 824 908**
**DE-A- 2 838 184**
**DE-A- 3 116 395**
**DE-B- 2 535 073**
**FR-A- 2 065 046**
**US-A- 2 412 209**

(73) Patentinhaber : **BASF Aktiengesellschaft**
**Carl-Bosch-Strasse 38**
**W-6700 Ludwigshafen (DE)**

(72) Erfinder : **Schroeder, Wolfgang, Dr.**
**Seebacher Strasse 51**
**W-6702 Bad Duerkheim (DE)**
Erfinder : **Voges, Dieter, Dr.**
**Richard-Wagner-Strasse 28**
**W-6800 Mannheim (DE)**

EP 0 057 884 B2

## Beschreibung

Die Erfindung betrifft ein Verfahren zur Herstellung von permethylierten Oligoaaminen und Aminoalkanolen (insbesondere z. B. von N,N,N′,N′-Tetramethyldiaminoalkanen und -oxaalkanen) durch Umsetzen entsprechender Diole bzw. Aminoalkanole über die Zwischenstufe der entsprechenden N,N-Dimethylaminoalkanole, mit Dimethylamin in der Gasphase an einem fest angeordneten Katalysator, vorzugsweise bei atmosphärischem Druck.

Aus der DE-A-2 535 073 ist bekannt, tertiäre aliphatische Monoamine durch Umsetzung von aliphatischen Alkoholen oder Aldehyden mit primären oder sekundären Aminen in Gegenwart von Kobalt- und/oder Kupferkatalysatoren in der Flüssigphase herzustellen.

In der US-A-2 412 209 wird die Herstellung von Aminoalkoholen durch Umsetzung von Glykolen mit Ammoniak in Gegenwart von Hydrierkatalysatoren (z.B. Raney-Nickel, Kupferchromit, Kupfer-/Nickelchromit, Eisen, Kobalt, Titan oder Kupfer) beschrieben.

Die EP-A-6454 lehrt die Herstellung von peralkylierten mehrwertigen Aminen durch Reaktion eines Diols mit einem sekundären Amin in der Flüssigphase bei einem Wasserstoffdruck von über 5 bar und in Gegenwart eines Katalysators, der als wesentliche aktive Bestandteile Kupfer und Chrom enthält.

Es ist weiterhin bekannt, Monoalkohole bzw. die entsprechenden Carbonylverbindungen bzw. Ketone mit Dimethylamin umzusetzen. Verfahren dieser Art sind beschrieben in der DE-A-2 709 864 und DE-A-2 838 184. Dagegen bereitete es bisher offenbar unüberwindliche Schwierigkeiten, diese an sich interessante Reaktion auf Diole bzw. N,N-Dimethylaminoalkanole anzuwenden, denn bisher wurden zur Herstellung der N,N,N′,N′-Tetramethyldiaminoalkane und -oxaalkane andere Wege beschritten. In der DE-B-1 795 762 wird die Umsetzung von Bis-(2-chlorethyl)-ether mit Dimethylamin und die Umsetzung von N,N-Dimethyl-1-amino-2-chlorethan mit Natrium-N,N-dimethylaminoethanolat beschrieben. Es wurde auch versucht, durch Umsetzung der Diamine mit Formaldehyd und anschliessende Hydrierung zum Ziel zu gelangen.

Diese Verfahren haben eine Reihe von Nachteilen. Entweder werden toxikologisch bedenkliche Stoffe verwendet, oder das Endprodukt enthält zu viel unvollständig methyliertes Diamin. Um zum reinen Endprodukt zu gelangen, sind also bei diesen Verfahren erhebliche Aufwendungen und eine Reihe von Verfahrensschritten notwendig.

Es wurde gefunden, dass man permethylierte Oligoamine und Aminoalkanole mit guter Ausbeute erhält, wenn man entsprechende N,N-Dimethylaminoalkanole oder Diole oder deren Gemische in der Gasphase mit Dimethylamin an kupferreichen Katalysatoren, die Oxide der aluminiums enthalten umsetzt. Als Diole sind sowohl reine Alkandiole geeignet als auch Diole im weiteren Sinne, d.h. Verbindungen, deren Kohlenstottgerüst durch Heteroatome unterbrochen ist. Ethandiol, 1,4-Butandiol, 1,5-Pentandiol, 1,6-Hexandiol und 1,10-Decandiol einerseits und Diethylenglykol oder N-Methyldiethanolamin andererseits sind z.B. geeignet.

Mit Vorteil lässt man die Reaktion bei Atmosphärendruck oder wenig darüber bei einer Reaktortemperatur zwischen 100 und 200°C ablaufen, wobei zweckmässig ein Röhrenreaktor mit fest angeordnetem Katalysator verwendet wird. Ein Trägergas für die Reaktionspartner ist erforderlich, denn die Reaktionstemperatur liegt im allgemeinen unter dem Siedepunkt der Diole; es eignet sich besonders Wasserstoff.

Im allgemeinen benötigt man eine Trägergas menge zwischen dem 10- und 1000-fachen des Reaktionsraums, die ständig umgewälzt wird. Raumgeschwindigkeiten [h$^{-1}$] vom 50- bis 1000-fachen des Reaktionsraums können z.B. eingestellt werden. - Die im Gasstrom enthaltene Menge an Diol bzw. Dimethylaminoalkanol ist natürlich abhängig von deren Dampfdruck bei Reaktionsbedingungen. Man erzielt normalerweise Umsatzgeschwindigkeiten (Raum-Zeit-Ausbeuten) von 0,02 bis 0,5 Volumenteilen Diol (flüssig) bzw. Dimethylaminoalkanol je Volumeneinheit Reaktionsraum. - Die im Gasstrom einzuhaltende Dimethylamin-Menge ergibt sich aus der Stöchiometrie. Bevorzugt wird ein gewisser Überschuss an Dimethylamin angewendet, so dass je Moläquivalent OH-Gruppen 1 bis 3, besonders 1 bis 1,5 mol Dimethylamin anwesend sind. Das Dimethylamin verlässt den Reaktor und das angeschlossene Kondensationssystem auf 3 verschiedenen Wegen:

1) durch chemische Reaktion
2) in gelöster Form im Reaktionsprodukt
3) gasförmig im Kreisgas.

Das den Weg 1 nehmende Dimethylamin wird durch frisches ersetzt. Das den Weg 2 gehende Dimethylamin wird bei der an sich bekannten Aufarbeitung des Reaktionsproduktes zurückgewonnen und wird erneut dem Reaktor zugeführt.

Bei den zu vervendenden kupferreichen, Katalysatoren, die Oxide des Aluminiums, soll der Kupferanteil zweckmässig über 20 Gew.% betragen.

Die Reaktionstemperatur liegt im Bereich zwischen 100 und 200 °C. Im unteren Bereich wird die Reaktion langsam und unvollständig und hört unter 100 °C praktisch ganz auf. Im oberen Bereich bilden sich zunehmend

2

Nebenprodukte, so dass wegen zunehmender Verluste and wachsender Schwierigkeiten bei der Abtrennung der Nebenprodukte die Herstellung der gewünschten Produkte allmählich unwirtschaftlich wird.

Zur praktischen Durchführung des Verfahrens werden die Reaktionsteilnehmer am besten zusammen mit dem als Trägergas dienenden Wasserstoff einem Verdampfer und dann dem Reaktor zugeführt.

Bevorzugt wird ein Röhrenreaktor, weil sich hiermit die Reaktionstemperatur am genauesten einstellen lässt. Im allgemeinen wird man den Katalysator innerhalb der Rohre anordnen, während der Raum zwischen den Rohren von einem Wärmeträgermedium durchströmt wird. Der Katalysator kann z.B. in Form zylindrischer Tabletten vorliegen, auch in Form von Extrudaten mit einem Durchmesser von einigen mm oder in anderer Form.

Da im frischen Katalysator das Kupfer i.a. zunächst in oxidischer Form vorliegt, muss es zur Aktivierung in den metallischen Zustand überführt werden. Dies geschieht in bekannter Weise durch ein Wasserstoff-Stickstoff-Gasgemisch z.B. bei einer Temperatur zwischen 120 und 250 °C.

Der Betriebsdruck ist vorteilhaft in der Nähe des atmosphärischen Druckes. Es wurde beobachtet, dass höherer Druck, z.B. schon 3 bar Überdruck, zur vermehrten Bildung von Nebenprodukten führt. Beispielsweise steigt der Anteil von N,N,N′-Trimethyldiaminoalkan im Reaktionsprodukt von einigen Zehntel Prozent auf über ein Prozent an.

Die Bildung des N,N,N′,N′-Tetramethyldiaminoalkans ist besonders bevorzugt, wenn als Reaktionspartner das entsprechende N,N-Dimethylaminoalkanol verwendet wird. In einigen Fällen ist dieses Zwischenprodukt (teilumgesetztes Produkt) durch eine unabhängige Reaktion günstig erhältlich. N,N-Dimethylaminoethanol ist z.B. durch Umsetzung von Ethylenoxid mit Dimethylamin leicht zu gewinnen. In anderen Fällen ist dieser Verbindungstyp nicht ohne weiteres zugänglich. Er entsteht jedoch bei der Reaktion des entsprechenden Diols mit Dimethylamin in jedem Falle als Zwischenprodukt und bildet bisweilen sogar das einzige Nebenprodukt.

Durch Einstellen passender Reaktionsbedingungen kann man erreichen, dass das N,N-Dimethylaminoalkanol als Reaktionsprodukt überwiegt. Man erreicht dies z.B. durch Absenken der Reaktionstemperatur, durch Verkürzen der Verweilzeit, geringeres Angebot an Dimethylamin oder durch Kombinieren dieser Massnahmen.

Das je nachdem als Haupt- oder Nebenprodukt gewonnene N,N-Dimethylaminoalkanol lässt sich durch Destillation bei vermindertem Druck in hoher Reinheit erhalten. Auch für dieses Produkt gibt es Verwendungsmöglichkeiten, z.B. bei der Herstellung bestimmter Färbstoffe. Im allgemeinen wird man die teilumgesetzten Produkte mit einer geeigneten Menge des entsprechenden Diols vermischen und erneut über den Verdampfer dem Reaktor zuführen. Auf diese Weise erzielt man eine hohe Ausbeute - im allgemeinen über 70% - an N,N,N′,N′-Tetramethyldiaminoalkan.

Beispiel 1

Herstellung von N,N,N′,N′-Tetramethyl-1,6-diaminohexan aus Hexandiol und Dimethylamin

Als Reaktor diente ein zylindrisches Rohr mit einem Durchmesser von 40 mm und einer Länge von 2500 mm. Das mit Katalysator gefüllte Volumen betrug 3000 cm³. Das Rohr war umgeben von einem Mantel, der von einem Wärmeträger durchflossen war. Oberhalb des Reaktors befand sich der Verdampfer. Er war ebenso konstruiert wie der Reaktor, hatte jedoch eine Länge von nur 700mm und war mit Raschigringen aus Metall gefüllt. Sein Mantelraum wurde vom gleichen Wärmeträger durchflossen wie der des Reaktors.

Unterhalb des Reaktors befand sich ein Wasserkühler, ein erster Abscheider, ein Solekühler und ein zweiter Abscheider. Die bei - 10 °C kondensierbaren Anteile des Reaktionsproduktes wurden hier flüssig abgeschieden, während das Trägergas mit Hilfe einer Gasumwälzpumpe zum Verdampfer zurückgefördert wurde. Am Verdampfereingang wurden dem Kreisgas Dimethylamin und Hexandiol zugefügt, die vollständig verdampften. Da das Hexandiol einen Schmelzpunkt von über 40°C hat, wurde es der Einfachheit halber als 75-prozentige methanolische Lösung verwendet.

Von dieser Lösung wurden stündlich 200 cm³ verdampft. Das Kreisgas enthielt ca. 20 Vol.-% Dimethylamin. Insgesamt betrug die Kreisgasmenge 400 Nl/h.

Als Katalysator diente der in der DE-PS 2 445 303 beschriebene Kupfer-AluminiumoxidKatalysator in Form zylindrischer Tabletten von 4mm Durchmesser, der bei 140 bis 200°C mit einem Gemisch aus 10 Vol.-% Wasserstoff und 90 Vol.-% Stickstoff reduziert worden war.

Die Umsetzung verläuft bei 160°C. Die in den Abscheidern angefallenen Kondensate wurden vereinigt, gaschromatographisch analysiert und fraktioniert: Ohne Berücksichtigung des Methanols, Dimethylamins und des entstandenen Wassers bestand das Kondensat aus 90% N,N,N′,N′-Tetramethyl-1,6-diaminohexan, 2% N,N,N′-Trimethyl-1,6-diaminohexan und 8% anderer Nebenprodukte. Die fraktionierte Destillation lieferte ein hochreines Produkt.

Beispiel 2

Herstellung von N,N,N′,N′-Tetramethyl-1,5-diamino-3-oxapentan

Der Anlage und dem Katalysator des Beispiels 1 wurden stündlich 150 cm³ eines Gemisches zugeführt, das aus gleichen molaren Anteilen Diethylenglykol und N,N-Dimethylamino-3-oxapentan-5-ol bestand.

Das Kreisgas enthielt 20 Vol.-% Dimethylamin. Die gesamte Kreisgasmenge betrug 600 Nl/h.

Die Umsetzung am Katalysator wurde bei 145 °C durchgeführt.

Die gesammelten Kondensate wurden gaschromatographisch analysiert und durch fraktionierte Destillation aufgearbeitet. Ohne Berücksichtigung von Dimethylamin und Wasser bestand das Kondensat aus 30 Gew.% N,N,N′,N′-Tetramethyl-1,5-diamino-3-oxapentan, 63 Gew.% N,N-Dimethylamino-3-oxapentan-5-ol und 7 Gew.% Nebenprodukten.

Die fraktionierte Destillation lieferte ein hochreines Produkt.

Beispiel 3

Herstellung von N,N,N′,N′-Tetramethyldiaminoethan

Man verfuhr wie in Beispiel 1 beschrieben.

Dem Verdampfer wurden stündlich 150 cm³ N,N-Dimethylaminoethanol zugeführt. Das Kreisgas enthielt 8 Vol.-% Dimethylamin. Die gesamte Kreisgasmenge betrug 600 Nl/h.

Die Umsetzung wurde bei 150°C vorgenommen. Die in den Abscheidern gesammelten Kondensate wurden wiederum analysiert und fraktioniert.

Ohne Berücksichtigung von Dimethylamin und Reaktionswasser bestand das Kondensat aus 36 Gew.-% N,N,N′,N′-Tetramethyldiaminoethan, 58 Gew.-% N,N-Dimethylaminoethanol und 6 Gew.% Nebenprodukten.

Fraktionierte Destillation und Trocknung lieferten ein hochreines Produkt.

## Patentansprüche

1. Verfahren zur Herstellung von permethylierten Oligoaminen und Aminoalkanolen durch Umsetzung von Diolen, Etherdiolen, N,N-Dimethylaminoalkanolen und/oder Azaalkanolen mit Dimethylamin, gegebenenfalls über die Zwischenstufe des N,N-Dimethylaminoalkanols, unter hydrierend/aminierenden Bedingungen, dadurch gekennzeichnet, dass man die Umsetzung in der Gasphase bei im wesentlichen atmosphärischem Druck und bei einer Temperatur zwischen 100 und 200°C an kupferreiden Katalysatoren, die Oxide der Aluminiums, enthalten vornimmt.

2. Verfahren nach Anspruch 1, dadurch gekennzeichnet, dass man Wasserstoff als Trägergas im Kreis führt und die Reaktionsprodukte aus dem Kreisgasstrom durch Kühlung gewinnt.

## Claims

1. A process for the preparation of permethylated oligoamines and aminoalkanols by the reaction of a diol, an ether diol, an N,N-dimethylaminoalkanol or an azaalkanol with dimethelamine, with or without the intermediate stage of the N,N-dimethylaminoalkanol, under conditions of hydrogenation/amination, wherein the reaction is carried out in the gas phase under essentially atmospheric pressure, at from 100 to 200°C, over a copper-rich catalyst containing an aluminum oxide.

2. A process as claimed in claim 1, wherein hydrogen is circulated as the carrier gas, and the reaction products are obtained from the circulating gas stream by cooling.

## Revendications

1. Procédé de préparation d'amino-alcanols et d'oligo-amines perméthylés par réaction de diols, d'étherdiols, de N,N-diméthyl-amino-alcanols et(ou) d'aza-alcanols avec la diméthyl-amine dans des conditions hydrogénantes/aminantes, en passant éventuellement par le stade intermédiaire du N,N-diméthyl-amino-alcanol, caractérisé en ce que la réaction est réalisée en phase gazeuse, à une température comprise entre 100 et 200°C et sous une pression sensiblement égale à la pression atmosphérique, en présence d'un catalyseur riche en cuivre qui contient un oxyde d'aluminium.

2. Procédé suivant la revendication 1, caractérisé en ce que l'on emploie comme gaz porteur de l'hydrogène circulant en circuit fermé, les produits réactionnels étant soutirés du circuit gazeux Par refroidissement.